Europäisches Patentamt

European Patent Office

Office européen des brevets

(19)

(11)　EP 0 748 894 B2

## (12)　NEW EUROPEAN PATENT SPECIFICATION

(45) Date of publication and mention
of the opposition decision:
**01.12.2004　Bulletin 2004/49**

(45) Mention of the grant of the patent:
**08.05.2002　Bulletin 2002/19**

(21) Application number: **96660027.2**

(22) Date of filing: **10.06.1996**

(51) Int Cl.7: **D06M 23/16**, A61L 15/52,
A61L 15/48, D04H 1/42

(54) **Method for increasing directionality of fluid transport in nonwoven sheet materials, and disposable absorbent articles containing the nonwoven material**

Verfahren zur Verbesserung der Strömungsführung von Flüssigkeiten in Vliesen, und absorbierende Wegwerfartikel enthaltend die Vliesen

Procédé pour améliorer le guidage des fluides dans les non-tissés et articles absorbants jetables contenant les non-tissés

(84) Designated Contracting States:
**AT BE CH DE DK ES FI FR GB GR IE IT LI LU MC NL PT SE**

(30) Priority: **14.06.1995　FI　952927**

(43) Date of publication of application:
**18.12.1996　Bulletin 1996/51**

(73) Proprietor: **Suominen Nonwovens Ltd.**
**29250 Nakkila (FI)**

(72) Inventor: **Hautojärvi, Joni**
**29250 Nakkila (FI)**

(74) Representative: **Haimelin, Jukka Ilmari et al**
**Oy Jalo Ant-Wuorinen Ab**
**Iso Roobertinkatu 4-6 A**
**00120 Helsinki (FI)**

(56) References cited:
**EP-A- 0 493 728**　　　　**DE-A- 2 105 019**
**DE-A- 4 130 343**　　　　**US-A- 5 213 881**

EP 0 748 894 B2

## Description

This invention relates to a method to treat nonwoven sheet or web materials with the purpose of amending the fluid transfer capacity of the nonwoven material in one plane direction of the sheet or web in relation to the transfer capacity in a plane direction crossing said one direction.

The following text considers the web or machine direction of the nonwoven material as the direction in which the fluid transfer capacity of the material is achieved higher than in a crossing direction.

Disposable hygienic absorbent articles, like diapers and sanitary napkins, are usually constructed with a fluid permeable coverstock of nonwoven sheet or a perforated film topsheet, a superabsorbent polymer and/or fluff pulp containing absorbent layer and a fluid impermeable backsheet. One problem related to diapers and sanitary napkins is the uneven distribution of fluid in the absorbent layer. The shape of diapers and sanitary napkins is elongated, and body fluid insulted into the central area of a disposable absorbent product wets only the center of the absorbent layer in the product. The edges of the absorbent layer, especially in the elongated direction, remain dry. Thus significant amount of the absorbent capacity of the absorbent layer is not used effectively. In addition, the wet superabsorbent polymer in the absorbent layer swells forming a gel and prevents further spreading of the fluid. At present, the trend is to develop thinner disposable absorbent product structures, and thus the aim is to reduce the amount of absorbent material in the products. This requires the effective use of the absorbent capacity of the disposable hygienic products.

Fluid can be distributed more uniformly into the absorbent layer by using a sublayer or a transport layer between the coverstock and the absorbent layer. The purpose of the transport layer is that it distributes fluid anisotropically, i.e. that the fluid transport rate in the elongated direction of the absorbent product is higher than its cross direction. One example of such a transport layer material is a nonwoven sheet material containing oriented hydrophilic fibres. The nonwoven sheet material is incorporated in the absorbent product with the oriented fibers in the elongation direction of the absorbent product. The carding method, which is well known in the art, normally orientates staple fibres to the machine direction in the nonwoven web. However, the slightly directioned fluid transport in nonwoven materials due to the oriented fibres is not always satisfying for the function of the nonwoven material as a transport layer.

The control of fluid transport direction in nonwoven materials is usually done by a fluid management member containing hydrophilic stripes or canals. US patent no. 4676786 illustrates a fluid transport layer which is constructed with a water repellent paper and a layer containing longitudinally oriented fluff pulp fibres. The paper has elongated openings, and thus the construction forms a transport layer with the hydrophilic stripes. Patent application publication no. WO 93/11727 presents a nonwoven coverstock connected with a sheet having hydrophilic V-shaped canals.

Considering the state of the art, particularly US patent specifications nos. 5314743 and 5353485 are referred. The patents comprise methods for producing nonwoven materials having anisotropic fluid transport properties. US patent no 5314743 discloses a nonwoven material being capable of intrafibre fluid transport. The fibres have eg. star- or cross-shaped cross sections, and therefore the fibres have intrafibre capillary structure for fluid transport.

In nonwoven materials the fibres can be oriented to a preferred direction in order to increase the directionality of fluid transport. US patent no. 5353485 discloses a method for producing a nonwoven material with non-uniform fibre composition. A layer of short hydrophilic fibres (fluff pulp) is formed on a staple fibre web. The web is hydroentangled on a wire containing an elongated element with larger diameter than the diameter of the wires in the wire. The wire separates the short fibres into hydrophilic stripes in the web under the hydroentanglement process. The hydrophilic stripes being constituted of short fibres orientate the fluid transport in the nonwoven material.

It is desirable to treat a conventional nonwoven material for transporting fluid anisotropically without affecting the structure of the material. Generally described, the present invention provides a process for post-treatment of nonwoven materials. The materials are treated with substances, i.e. finishing agents, to form parallel hydrophilic and hydrophobic or semihydrophobic stripes on the nonwoven material surface. The parallel stripes guide fluid transport in the nonwoven materials to the elongated direction of the stripes. The method according to the invention is cost-effective and can be connected on-line in a nonwoven web manufacturing process. The method can be applied for thermobonded nonwovens, hydroentangled nonwovens, spunbonded nonwovens, meltblown nonwovens and other nonwovens produced by the processes well known in the art.

The objects, features and advantages of the present invention will become fully apparent from the appended claims.

One preferred embodiment of the present invention is to treat the nonwoven material on a flexographic printing machine, which is well known in the art of conventional color printing technology. The color pigment solution or dispersion in the printing machine is replaced with a hydrophilic or hydrophobic finishing agent liquid. According to the invention, the nonwoven material is zone-treated with the finishing agent so, that after the treatment the material contains finishing agent stripes on the treated surface. The stripes can be either hydrophilic or hydrophobic, and they are preferably oriented to the elongated direction (machine direction) of the nonwoven material web. Hydrophobic nonwoven materials are preferably zone-treated with a 10-80 wt-% wetting agent water dispersion. Hydrophilic nonwoven materials are preferably zone-treated with a 10-80 wt-% hydrophobizing agent water dispersion. Correspondingly, semihydrophobic nonwoven materials can be treated either with a wetting agent or with a hydrophobizing agent, or with both type of the finishing agents. Organic solvent based finishing agent solutions can also be used, if faster drying of the nonwoven material in the treatment process is required. The surface of the zone-treated nonwoven material is consisted of hydrophilic stripes, which orientate fluid transport to the elonged direction of the stripes, and of hydrophobic stripes, which prevent fluid transport to the cross direction of the stripes.

The advantage of the above described preferred embodiment of the present invention is the accurate targeting of the finishing agents to the precisely defined zones on the surface of the nonwoven material. Targeted hydrophilicity or hydrophobicity on the nonwoven material surface does not require any structural modification of the surface. In addition, the flexographic printing method gives the possibility for simple and inexpensive modification of the width and the shape of the finishing agent stripes according to the specific end-use requirements for the nonwoven material in absorbent articles.

The finishing agent stripes can be applied to the surface of the nonwoven materials also with a patterned coating roll or with targeted sprays. However, these methods do not enable as good control of the applied quantity of the finishing agents and of the precise targeting of the stripes to the surface of the nonwoven material.

In the enclosed drawing,

Figure 1 is a diagrammic drawing of the surface of a nonwoven material treated according to the invention.
Figure 2 is a diagrammic cross-sectional drawing of a flexographic printing machine used to apply a targeted coating to the surface of a nonwoven material.
Figure 3 is a graph showing the effect of various stripe-shaped hydrophilic zone treatments to the distribution ratio of fluid in a carded, hydrophobic thermobonded nonwoven material.
Figure 4 is a graph showing the effect of various stripe-shaped hydrophobic zone treatments to the distribution ratio of fluid in a carded, hydrophilic thermobonded nonwoven material.
Figure 5 is a graph showing the effect of various stripe-shaped hydrophilic zone treatments to the distribution ratio of fluid in a carded, semihydrophobic hydroentangled nonwoven material
Figure 6 is a graph showing the effect of various stripe-shaped permanent hydrophilic zone treatments to the distribution ratio of fluid in a carded, hydrophobic thermobonded nonwoven material.
Figure 7 is a graph showing the effect of various stripe shaped permanent hydrophilic zone treatments to the distribution ratio of fluid in a carded, semihydrophobic hydroentangled nonwoven material.

While the invention will be described according to a preferred embodiment of the invention, it is not the purpose to limit the invention to that embodiment. All alternatives and modifications of the present invention are defined by the appended claims.

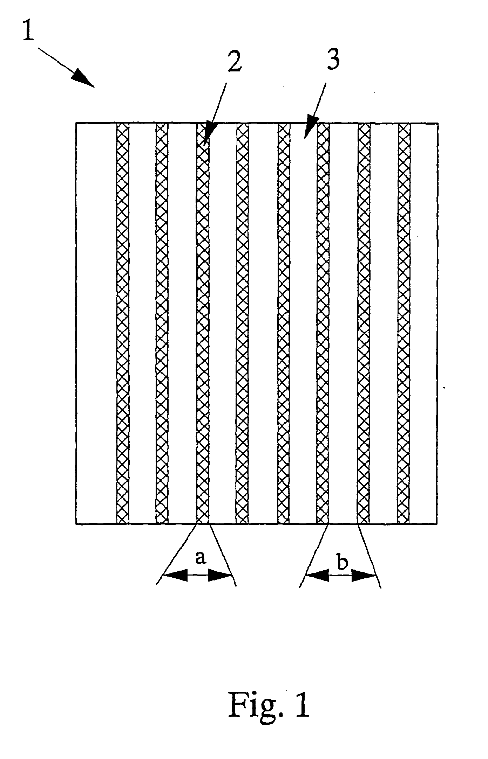

A nonwoven material, which is zone-treated according to the invention, is shown in figure **1**. The nonwoven material **1** can be composed of staple fibres, like polyolefin fibres, viscose fibres, polyester fibres or combinations of said fibres, or the material can be composed of polymer filaments. The nonwoven material **1** can be hydrophilic, hydrophobic or semihydrophobic, and the basis weight of the material can be in the range of 10 - 150 g/m$^2$. The nonwoven material **1** is produced by thermo-bonding, spunbonding, meltblowing or hydroentanglement process, or by any other process well-known in the art. Hydrophobic nonwoven materials are composed of fibres having water contact angle more than 90°. Similarly, hydrophilic nonwoven materials are composed of fibres having water contact angle less than 90°. Semihydrophobic nonwoven materials contain both hydrophobic and hydrophilic fibres.

The zone-treated nonwoven material **1**, shown in figure 1, contains stripes **2** formed of a finishing agent on the surface of the material. The stripes **2** having width "**a**" are oriented to the elongated direction (machine direction) of the nonwoven material web. The stripes **2** are either hydrophilic or hydrophobic. The stripes on the surface of a hydrophobic nonwoven material are preferably hydrophilic, and thus the material is printed with a wetting agent water dispersion. The unprinted gaps **3**, having width "**b**", between the stripes 2 remain thus hydrophobic. Correspondingly, the stripes on the surface of a hydrophilic nonwoven material are preferably hydrophobic, and the material is printed with a hydrophobizing agent water dispersion. The gaps **3** remain thus hydrophilic. Either hydrophilic or hydrophobic stripes can be applied to the surface of a semihydrophobic material with semihydrophobic unprinted gaps between the stripes.

One embodiment of the invention is to print the stripes with orientation to the cross direction of the nonwoven material web. However, more preferably the stripes are printed to the direction of the fibre orientation (machine direction) of the nonwoven web, as the fibres orientate fluid transport to the direction of the fibre orientation. In this case, both the fibre orientation and the stripe orientation direct the transport of fluid to the same direction in the nonwoven material. Nonwoven webs laid by carding method have usually fibre orientation to the machine direction of the web. Thus nonwoven materials produced by carding process are most preferably printed with stripes having the orientation to the machine direction of the material.

Organomodified poly(alkyl oxide) derivatives of poly(dimethyl siloxanes) can be used as wetting agents. The water solubility of siloxane based surfactants is controlled by the length of the poly(alkyl oxide) chains and by the substitution degree of the poly(dimethyl siloxane). The advantage of the organomodified poly(dimethyl siloxanes) is the low surface energy on polyolefin materials, which improves the spreading of the siloxanes to the surface of polyolefin fibres. Poly(dimethyl siloxanes) can be modified in the way, that the siloxane based surfactant is both hydrophilic and water insoluble. By using water insoluble wetting agents, it is possible to hydrophilize nonwoven materials more permanently than with water soluble wetting agents, which are washed off easily from the fibre surface.

Hydrophobizing agents are preferably water insoluble paraffin compounds. Due to the high viscosity of said paraffin compounds, hydrophobizing agent water dispersions may require heating to ensure the even spreading of the compound to the nonwoven surface. Thus, more preferably low-viscosity poly(dimethyl siloxane) oils, or water dispersions of said oils, are used as hydrophobic finishing agents in the zone-treatment method. The surface tension of silicone oils is also lower than the surface tension of paraffin compounds.

Fluid is transported in nonwoven materials mostly in the capillaries, which are formed by the porous structure of the nonwoven materials. The fluid transport rate is proportional to the capillary pressure. The capillary pressure for cylinder-shaped capillaries can be expressed by the equation below, which is well known in the art of surface science.

$$P = \frac{2\gamma\cos\theta}{r}$$

where P is the capillary pressure, $\gamma$ is the surface tension of the fluid, $\theta$ is the fluid contact angle on the interface between the fluid and the capillary wall and r is the radius of the capillar.

The function of the preferred embodiment of the current invention is based on the differencies between the fluid transport rates in the hydrophilic stripes and the hydrophobic stripes. The fluid transport rate is higher in the hydrophilic pore structure in the hydrophilic stripes having water contact angle less than 90°, than in the hydrophobic pore structure of the hydrophobic stripes having water contact angle over 90°. Due to the alternating hydrophitic/hydrophobic structure across to the cross direction of the nonwoven material, fluid is transported to the elongated direction (machine direction) of the nonwoven material along the continuous hydrophilic stripes, while the continuous hydrophobic stripes are preventing fluid transport to the cross direction of the nonwoven material.

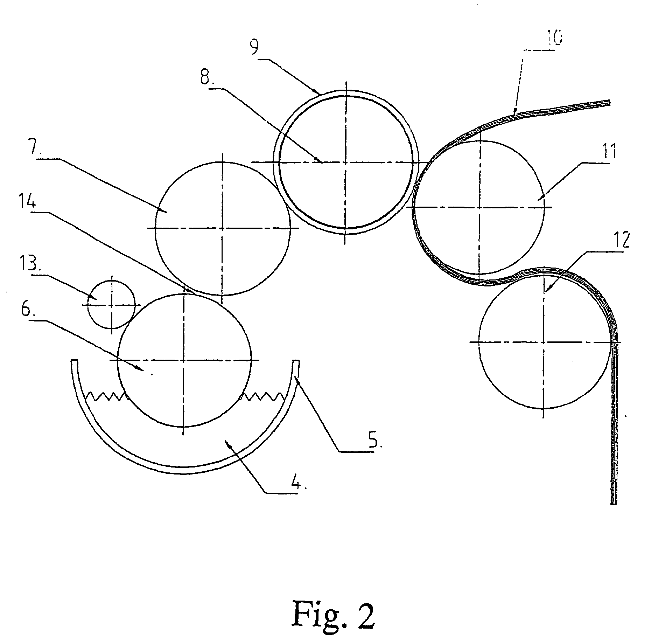

The finishing agent stripes are printed to the surface of the nonwoven material with the flexographic printing method shown in figure 2. The flexographic printing method described here is well known in the art of conventional colour printing. The printing method used in the invention is not limited to the transfer roll flexographic printing method described here. Also other variations or alternatives of the flexographic printing method are suitable for the surface zone treatment of the nonwoven material.

The finishing agent solution or dispersion **4** is transferred from the printing liquid container **5** to the screened anilox roll **7** by the transfer roll **6**, which is in contact with the anilox roll **7**. The anilox roll **7** wets the printing plate **9** with the printing liquid **4**. The printing plate **9** is fixed to the printing roll **8**. The printing plate **9** is preferably a photopolymer containing printing plate having desired embossed stripe pattern exposed to the plate. The printing plate **9** surrounding the printing roll **8** transfers the finishing agent solution or dispersion **4** to the surface of the nonwoven material **10**. The printing liquid **4** forms the finishing agent stripes, corresponding the pattern of the printing plate **9**, to the nonwoven material **10**. The impression roll **11** and the nip roll **12** guide the running of the nonwoven material **10** in the printing machine. The amount of the finishing agent solution or dispersion **4** transferred to the nonwoven material **10** is controlled by the pressure between the scraping roll **13** and the transfer roll **6**. The amount of the liquid **4** transferred to the material **10** can also be controlled by the size of the nip **14** between the transfer roll **6** and the anilox roll **7**.

A water soluble surfactant dissolves to the fluid insulted to the nonwoven material thus lowering the surface tension of the fluid. This phenomenon promotes the fluid penetration into the hydrophobic stripes of the nonwoven material, which reduces the transport of the fluid to the direction of the hydrophilic stripes. Thus, the hydrophilic finishing agent is a water dispersible, water insoluble hydrophilic organomodified poly(dimethyl siloxane) wetting agent, or some other water insoluble wetting agent, which hydrophilizes the nonwoven material more permanently than a water soluble wetting agent does. The water insoluble wetting agent does not significantly dissolve in the fluid insulted in the nonwoven material, and thus the fluid transport properties of the nonwoven material remain even when the material is wet.

The active content of the finishing agent printing solutions or dispersions is preferably between about 5 and 80 percent, and most preferably about 20 percent by weight of the solution. The amount of the finishing agent remaining in the nonwoven material in the zone-treatment process is preferably between 0.1 and 2 percent, and most preferably about 0.5 percent by weight of the nonwoven material. The finishing agent content of the nonwoven material depends on the ratio of the area covered by the finishing agent stripes on the nonwoven material surface divided by the total surface area of the nonwoven material.

The hydrophilic finishing agent stripes are preferably applied to the surface of hydrophobic nonwoven materials in the way, that the untreated side of the nonwoven materials remains hydrophobic. This requires, that the printing liquid does not wet thoroughly the nonwoven material. The treatment process is most surface sensitive, when finishing agent water dispersions are used for printing hydrophobic nonwoven materials.

The nonwoven materials according to the invention are preferably used as sublayers or transport layers in disposable, hygienic absorbent articles, or as a coverstock material functioning both as a fluid pervious coverstock and as a fluid distributing transport layer. The purpose of such a sublayer or transport layer is to distribute fluid, which is insulted through the coverstock, evenly to the absorbent core in the absorbent article. The preferred ratio of the hydrophilic area divided by the hydrophobic area on the nonwoven material surface depends on the end-use of the zone-treated nonwoven material. For the sublayer use eg. in feminine hygienic pads, different width of hydrophilic and hydrophobic stripes are needed for the optimal performance than in baby diapers, where bigger amounts of fluid are absorbed.

The following examples illustrate, but not limit, the function of the invention concerning fluid distribution in zone treated nonwoven materials. The fluid spreading test methods used in the examples were the following:

**Method 1.** 20 cm x 20 cm piece of the test nonwoven sheet material was cut and put on a plexiglass plate. 1 ml of coloured saline solution (comprising 9 g sodium chloride and 400 mg red erythrocine in 1 l distilled water, surface tension 71 mN/m) was insulted to the hydrophilic central area of the nonwoven piece. After 1 minute, the test piece was removed and put on a white sheet of paper. The spreading of the coloured solution in the test piece was measured in the elongated direction and in the cross direction of the nonwoven material. The reported value is the average of three measurements.

**Method 2.** 3 pieces of 125 mm x 125 mm blotting paper (Postlip papers, medium white W/S, supplier Hollingsworth & Vose Co., Ltd.) was put on a plexiglass plate. 20 cm x 20 cm piece of the test nonwoven sheet material was laid on the paper layer. The test piece was covered with a 20 cm x 20 cm piece of a nonwoven coverstock material (20 g/m$^2$ hydrophilic, thermobonded polypropylene nonwoven). 2 ml of the coloured saline solution was insulted to the central area of the coverstock material. After 1 minute the coverstock material was removed, and the test piece was put on a white sheet of paper. The spreading of the coloured solution in the test piece was measured in the elongated direction and in the cross direction of the nonwoven material. The reported value is the average of three measurements.

**Method 3.** 10 cm x 15 cm piece of a superabsorbent polymer containing dryformed paper was put on a 20 cm x 15 cm (lengh x width) plexiglass plate. 10 cm x 15 cm piece of the nonwoven test material sheet was laid on the superabsorbent paper. A testing device was put on the test material. The device was constructed of a 20 cm x 17 cm x 0.4 cm (length x width x thickness) plexiglass plate with a 0.4 cm diameter hole in the center. An open plexiglass cylinder (length 7 cm, inner diameter 3 cm) was secured vertically on the plate coinciding with the hole. 20 ml of the coloured saline solution was insulted into the cylinder. After half a minute, the test device was removed, and the spreading of the saline solution was measured in the test piece similarly to the previous examples. The reported value is the average of three measurements.

**Method 4.** Similar to method 3, except a 15 cm x 20 cm piece of a nonwoven coverstock material (20 g/m$^2$ hydrophilic, thermobonded polypropylene nonwoven) was used between the test material and the testing device. The reported value is the average of three measurements.

**Finishing agent content.** The amount of the finishing agent applied in the zone treatment process to the nonwoven material was analyzed by the following way: 1 g of the nonwoven material was extracted with 5 ml of sulphur carbon. The concentration of the finishing agent (organomodified poly(dimethyl siloxane) ) in the extract was measured with IR spectrophotometer with the absorption wave number of Si - O bond (1259 cm$^{-1}$). The reported value is the average of two measurements.

Methods 1 and 2 illustrate the fluid distribution properties of the nonwoven materials according to the invention with small amounts of fluid (simulate the function of the materials eg. in feminine hygiene pads). Correspondingly, methods 3 and 4 illustrate the fluid distribution properties of the nonwoven materials with higher amounts of fluid (simulate the function of the materials eg. in baby diapers). Methods 2 and 4, where a piece of a coverstock material was used on the nonwoven test material, illustrate especially the function of the nonwoven material as a nonwoven sublayer or a fluid transport layer in absorbent articles. The fluid distribution ratio can be expressed by dividing the spreading of the test fluid in the elongated direction (MD, machine direction) by the spreading of the fluid in the cross direction (CD) in the nonwoven material. The higher the fluid distribution ratio is, the better is the performance of the nonwoven material according to the invention. The fluid distribution ratio between the MD spreading and the CD spreading is preferably over 2, and most preferably over 3 with the nonwoven materials according to the invention.

In the following, Examples 1 and 3 are reference Examples.

## Example 1

Carded, 100% polypropylene staple fibres containing, thermobonded 32 g/m$^2$ hydrophobic nonwoven material was zone-treated with hydrophilic stripes. The stripes were printed to the surface of the nonwoven material sheet with a "PAVEMA" flexographic printing machine (Maschinenbau Ludwig Meyer, Düsseldorf) with a transfer roll printing unit. The printing liquid used was 40 wt-% water solution of a water soluble nonionic wetting agent (organomodified poly

(dimethyl siloxane) ). The printing was performed with four different photopolymer printing plates with the following stripe widths (stripe width /gap width): 3 mm / 12 mm, 7 mm / 7 mm, 10 mm / 20 mm and 15 mm / 15 mm.

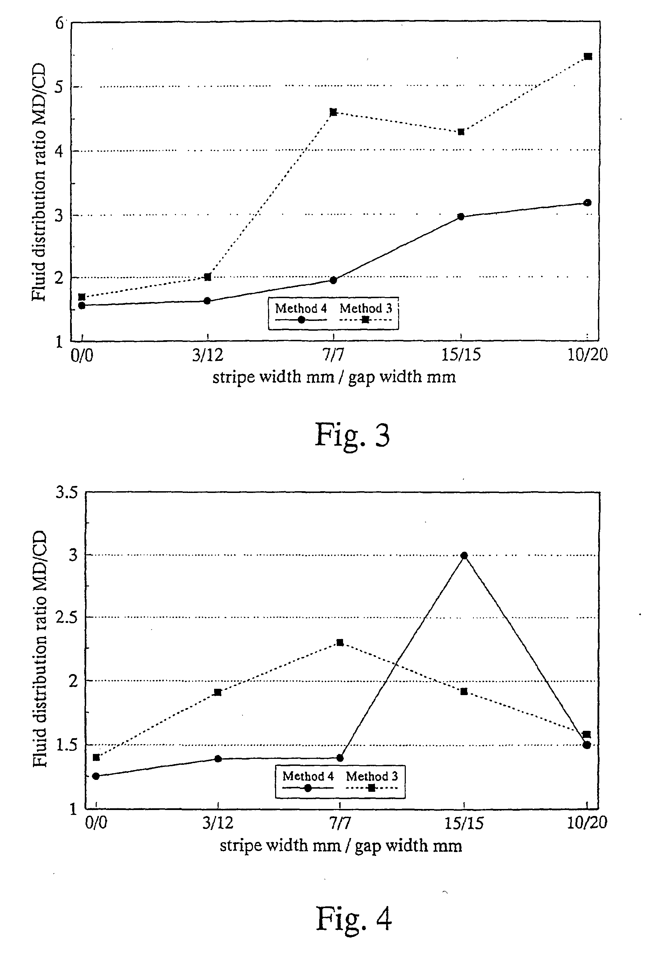

The fluid distribution ratio in the zone treated nonwoven materials and the finishing agent content of the materials were measured according to the methods described previously. The MD/CD fluid distribution ratio and the finishing agent content of the nonwoven materials are reported in table 1. In figure 3 is shown the MD/CD fluid distribution ratio of the nonwoven materials in the Y-axis measured with the methods 3 and 4, and the stripe width / gap width of the printing plates in the X-axis. The Y-axis value in the X-axis point 0/0 corresponds the value of the sample "PDMS1" in the comparative example 6.

**Example 2**

Carded, 100% polypropylene staple fibres containing, thermobonded 25 g/m$^2$ hydrophilic nonwoven material was zone-treated with hydrophobic stripes. The stripes were printed to the surface of the nonwoven material sheet as in example 1. The printing liquid used was 35 wt-% water dispersion of a hydrophobic poly(dimethyl siloxane) oil. The printing was performed with the four different photopolymer printing plates as in example 1.

The fluid distribution ratio in the zone treated nonwoven materials and the finishing agent content of the materials were measured according to the methods described previously. The MD/CD fluid distribution ratio and the finishing agent content of the nonwoven materials are reported in table 2. In figure 4 is shown the MD/CD fluid distribution ratio of the nonwoven materials in the Y-axis measured with the methods 3 and 4, and the stripe width / gap width of the printing plates in the X-axis. The Y-axis value in the X-axis point 0/0 comprises the value of the sample "Hfi" in the comparative example 6.

**Example 3**

Carded, 50 % polypropylene and 50% viscose staple fibres to combination by weight containing, hydroentangled 30 g/m$^2$ semihydrophobic nonwoven material was zone treated with hydrophilic stripes. The stripes were printed to the surface of the nonwoven material as in example 1. The printing liquid used was 40 wt% water solution of a water soluble nonionic wetting agent (organomodified poly(dimethyl siloxane)). The printing was performed with the four different photopolymer printing plates as in example 1.

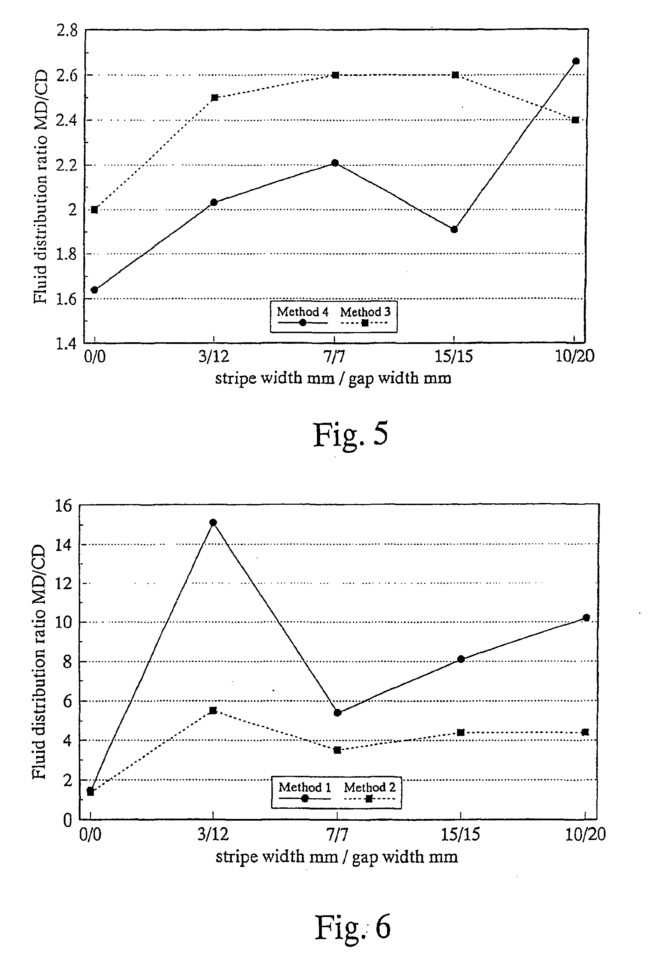

The fluid distribution ratio in the zone treated nonwoven materials and the finishing agent content of the materials were measured according to the methods described previously. The MD/CD fluid distribution ratio and the finishing agent content of the nonwoven materials are reported in table 3. In figure 5 is shown the MD/CD fluid distribution ratio of the nonwoven materials in the Y-axis measured with the methods 3 and 4, and the stripe width / gap width of the printing plates in the X-axis. The Y-axis value in the X-axis point 0/0 comprises the value of the sample "VN1" in the comparative example 6.

**Example 4**

The nonwoven material of example 1 was zone treated with hydrophilic stripes. The stripes were printed to the surface of the nonwoven material as in example 1. The printing liquid used was 40 wt-% water dispersion of a water insoluble nonionic wetting agent (ethoxylated poly(alkyl oxide) modified poly(dimethyl siloxane) ), which hydrophilizes the nonwoven material more permanently than a water soluble wetting agent does. The printing was performed with the four different photopolymer printing plates as in example 1.

The fluid distribution ratio in the zone treated nonwoven materials and the finishing agent content of the materials were measured according to the methods described previously. The MD/CD fluid distribution ratio and the finishing agent content of the nonwoven materials are reported in table 4. In figure 6 is shown the MD/CD fluid distribution ratio of the nonwoven materials in the Y-axis measured with the methods 3 and 4, and the stripe width / gap width of the printing plates in the X-axis. The Y-axis value in the X-axis point 0/0 comprises the value of the sample "PDMS2" in the comparative example 6.

**Example 5**

Carded, 50% polypropylene and 50% viscose staple fibres blend by weight containing, hydroentangled 50 g/m$^2$ semihydrophobic nonwoven material was zone treated with hydrophilic stripes. The stripes were printed to the surface of the nonwoven material as in example 1. The printing liquid used was 40 wt-% water dispersion of a water insoluble nonionic wetting agent (ethoxylated poly(alkyl oxide) modified poly(dimethyl siloxane) ), which hydrophilizes the nonwoven material more permanently than a water soluble wetting agent does. The printing was performed with the four different photopolymer printing plates as in example 1.

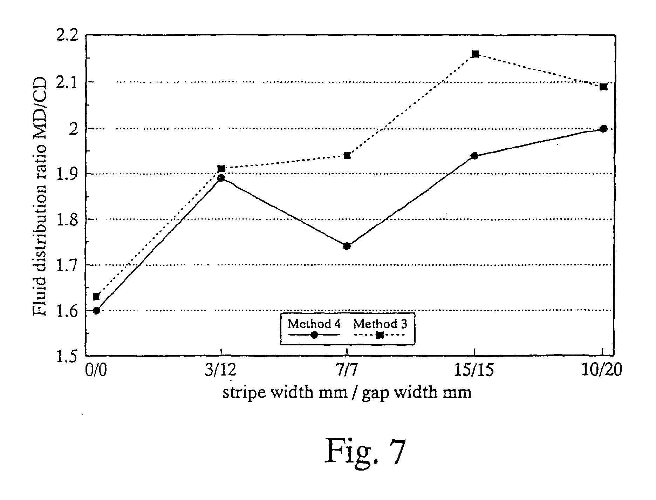

The fluid distribution ratio in the zone treated nonwoven materials and the finishing agent content of the materials were measured according to the methods described previously. The MD/CD fluid distribution ratio and the finishing agent content of the nonwoven materials are reported in table 5. In figure 7 is shown the MD/CD fluid distribution ratio of the nonwoven materials in the Y-axis measured with the methods 3 and 4, and the stripe width / gap width of the printing plates in the X-axis. The Y-axis value in the X-axis point 0/0 comprises the value of the sample "Hfi" in the comparative example 6.

**Comparative example 6**

The untreated nonwoven material of example 1 was treated uniformly with the water soluble wetting agent used in the same example (sample "PDMS1"). The untreated nonwoven material of example 4 was treated uniformly with the water insoluble wetting agent used in the same example (sample "PDMS2"). The total wetting agent content of the both samples was 1.0% by weight of the samples. The other comparative samples were the untreated nonwoven material of example 2 (sample "Hfi"), the untreated material of example 3 (sample "VN1"), and the material of example 5 (sample "VN2"). The fluid distribution tests were performed to all of the comparative samples as in the previous examples. The results are reported in table 6.

Table 1.

| Stripe mm / gap mm | Fluid spreading Method 1 | | | Fluid spreading Method 2 | | | Fluid spreading Method 3 | | | Fluid spreading Method 4 | | | Finish. agent content wt-% |
|---|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD | |
| 3/12 | 107 | 50 | 2.1 | 73 | 22 | 3.3 | 62 | 31 | 2.0 | 80 | 49 | 1.6 | 1.6 |
| 7/7 | 107 | 49 | 2.2 | 74 | 20 | 3.7 | 78 | 17 | 4.6 | 74 | 38 | 1.9 | 6.0 |
| 15/15 | 85 | 59 | 1.4 | 68 | 24 | 2.8 | 77 | 18 | 4.3 | 74 | 25 | 3.0 | 2.6 |
| 10/20 | 87 | 72 | 1.2 | 74 | 26 | 2.8 | 71 | 13 | 5.5 | 70 | 22 | 3.2 | 2.8 |

Table 2.

| Stripe mm / gap mm | Fluid spreading Method 1 | | | Fluid spreading Method 2 | | | Fluid spreading Method 3 | | | Fluid spreading Method 4 | | | Finish. agent content wt-% |
|---|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD | |
| 3/12 | 117 | 64 | 1.8 | 47 | 32 | 1.5 | 21 | 11 | 1.9 | 25 | 18 | 1.4 | 13 |
| 7/7 | 130 | 48 | 2.7 | 73 | 34 | 2.1 | 23 | 10 | 2.3 | 28 | 20 | 1.4 | 1.0 |
| 15/15 | 120 | 75 | 1.6 | 92 | 26 | 3.5 | 25 | 13 | 1.9 | 74 | 25 | 3.0 | 0.8 |
| 10/20 | 124 | 55 | 2.3 | 68 | 25 | 2.7 | 19 | 12 | 1.6 | 27 | 18 | 1.5 | 0.7 |

**Table 3.**

| Stripe mm / gap mm | Fluid spreading Method 1 | | | Fluid spreading Method 2 | | | Fluid spreading Method 3 | | | Fluid spreading Method 4 | | | Finish. agent content |
|---|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD | wt-% |
| 3/12 | 115 | 53 | 2.2 | 60 | 44 | 1.4 | 65 | 26 | 2.5 | 59 | 29 | 2.0 | 1.6 |
| 7/7 | 115 | 53 | 2.2 | 63 | 44 | 1.4 | 84 | 32 | 2.6 | 84 | 38 | 2.2 | 4.6 |
| 15/15 | 127 | 50 | 2.5 | 62 | 40 | 1.6 | 60 | 23 | 2.6 | 59 | 31 | 1.9 | 2.5 |
| 10/20 | 115 | 51 | 2.3 | 66 | 38 | 1.7 | 82 | 34 | 2.4 | 85 | 32 | 2.7 | 2.8 |

**Table 4.**

| Stripe mm / gap mm | Fluid spreading Method 1 | | | Fluid spreading Method 2 | | | Fluid spreading Method 3 | | | Fluid spreading Method 4 | | | Finish. agent content |
|---|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD | wt-% |
| 3/12 | 257 | 17 | 15.1 | 120 | 22 | 5.5 | 73 | 24 | 3.0 | 92 | 42 | 2.2 | 0.6 |
| 7/7 | 156 | 29 | 5.4 | 116 | 33 | 3.5 | 87 | 19 | 4.6 | 88 | 67 | 1.3 | 1.6 |
| 15/15 | 153 | 19 | 8.1 | 87 | 20 | 4.4 | 85 | 17 | 5.0 | 100 | 22 | 4.5 | 1.2 |
| 10/20 | 163 | 16 | 10.2 | 101 | 23 | 4.4 | 55 | 11 | 5.0 | 82 | 34 | 2.4 | 1.1 |

EP 0 748 894 B2

**Table 5.**

| Stripe mm / gap mm | Fluid spreading Method 1 | | | Fluid spreading Method 2 | | | Fluid spreading Method 3 | | | Fluid spreading Method 4 | | | Finish. agent content |
|---|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD | wt-% |
| 3/12 | 109 | 51 | 2.1 | 77 | 46 | 1.7 | 88 | 46 | 1.9 | 89 | 47 | 1.9 | 0.4 |
| 7/7 | 100 | 54 | 1.9 | 73 | 47 | 1.6 | 93 | 48 | 1.9 | 94 | 54 | 1.7 | 0.6 |
| 15/15 | 105 | 51 | 2.1 | 72 | 46 | 1.6 | 93 | 43 | 2.2 | 97 | 50 | 1.9 | 0.7 |
| 10/20 | 106 | 50 | 2.1 | 72 | 47 | 1.6 | 92 | 44 | 2.1 | 94 | 47 | 2.0 | 0.5 |

**Table 6.**

| Comparative sample | Fluid spreading Method 1 | | | Fluid spreading Method 2 | | | Fluid spreading Method 3 | | | Fluid spreading Method 4 | | |
|---|---|---|---|---|---|---|---|---|---|---|---|---|
| | MD/mm | CD/mm | MD/CD | MD/mm | CD/mm | MD/CD | MD/mm | CD/mm | MD/CD | MD/mm | CD/mm | MD/CD |
| Hfi | 111 | 69 | 1.6 | 67 | 43 | 1.6 | 35 | 25 | 1.4 | 30 | 24 | 1.3 |
| PDMS 1 | 87 | 73 | 1.2 | 67 | 47 | 1.4 | 73 | 43 | 1.7 | 66 | 42 | 1.6 |
| PDMS 2 | 87 | 59 | 1.5 | 59 | 43 | 1.4 | 64 | 46 | 1.4 | 58 | 40 | 1.5 |
| VN 1 | 119 | 54 | 2.2 | 65 | 41 | 1.6 | 57 | 29 | 2.0 | 61 | 37 | 1.6 |
| VN 2 | 100 | 56 | 1.8 | 70 | 46 | 1.5 | 78 | 48 | 1.6 | 69 | 43 | 1.6 |

EP 0 748 894 B2

**Claims**

1. A method to direct fluid transport in one plane direction of nonwoven sheet materials by means of parallel and continuous stripes achieved in the nonwoven sheet material, of which stripes at least one has a fluid transport ability different from that of the adjacent stripe, **characterized in that** in order to define a stripe of an amended fluid transport ability in the nonwoven sheet material, the respective area of the nonwoven sheet material is treated with a water dispersible, water insoluble substance affecting the fluid transport properties of the nonwoven material.

2. The method of claim 1, **characterized in that** the stripes are formed into two groups of alternating stripes, where the stripes belonging to the same of said groups have essentially equal fluid transport properties.

3. The method of claim 2, **characterized in that** the nonwoven material is treated with the substance in order to define the stripes in one of said stripe groups, while the fluid transport properties of the original nonwoven material persists for the stripes of the other group.

4. A method of any of the claims 1-3, **characterized in that** the stripes of amended fluid transport properties are applied to the nonwoven sheet material by printing technology, especially flexographic printing technology.

5. A nonwoven sheet or web material comprising:

   a first group of parallel stripes consisting of continuous hydrophilic stripes,
   a second group of stripes consisting of continuous hydrophobic stripes between the stripes of the first group, **characterized in that** at least the stripes in one of said groups being accomplished by means of a water dispersible, water insoluble substance affecting the fluid transport properties of the nonwoven material in the elongated direction and in the cross direction of the stripes.

6. The nonwoven material of claim 5, wherein said hydrophilic and/or hydrophobic stripes are printed on the nonwoven sheet material using printing liquid consisting of a water dispersion, or a solvent solution of the substanse affecting the fluid transport properties of the nonwoven material.

7. The nonwoven material of claims 5-6, **characterized in that** said nonwoven material is composed of polyolefin fibres, polyesther fibres, viscose fibres, or combinations of said fibres.

8. The nonwoven material of claims 5-7, **characterized in that** the basis weight of said material is between 10 g/m$^2$ and 150 g/m$^2$.

9. The nonwoven material of claims 5-8, **characterized in that** the rate of the fluid penetration in the elongated direction of said stripes is at least 1.8 times the rate of the fluid penetration in the cross direction of said stripes.

10. A disposable absorbent article being capable of absorbing discharged body fluids comprising at least:

    a fluid impervious outer cover layer;
    an absorbent layer;
    at least one layer of the nonwoven material of claims 5-9.

**Patentansprüche**

1. Verfahren zur Fluidtransportführung in Richtung einer Ebene von Vliesen mittels paralleler und kontinuierlicher Streifen in dem Vlies, von denen wenigstens ein Streifen ein anderes Fluidtransportvermögen als der benachbarte Streifen hat, **dadurch gekennzeichnet, daß** zur Abgrenzung eines Streifens mit einem geänderten Fluidtransportvermögen in dem Vlies der betreffende Bereich des Vlieses mit einer wasserdispersiven, wasserunlöslichen Substanz behandelt wird, die die Fluidtransporteigenschaften des Vlieses beeinflußt.

2. Verfahren nach Anspruch 1, **dadurch gekennzeichnet, daß** die Streifen in zwei Gruppen aus einander abwechselnden Streifen ausgebildet werden, wobei die zur selben Gruppe gehörenden Streifen weitgehend gleiche Fluidtransporteigenschaften haben.

3. Verfahren nach Anspruch 2, **dadurch gekennzeichnet, daß** das Vlies mit der Substanz behandelt wird, um die Streifen in einer der Streifengruppen abzugrenzen, wobei die Fluidtransporteigenschaften des ursprünglichen Vlieses bei den Streifen der anderen Gruppe beibehalten werden.

4. Verfahren nach einem der Ansprüche 1 bis 3, **dadurch gekennzeichnet, daß** die Streifen mit den geänderten Fluidtransporteigenschaften auf das Vlies durch ein Druckverfahren aufgebracht werden, insbesondere ein flexographisches Druckverfahren.

5. Vlies mit:

   einer ersten Gruppe aus parallelen Streifen, die aus kontinuierlichen hydrophilen Streifen bestehen,
   einer zweiten Gruppe aus Streifen, die aus kontinuierlichen hydrophoben Streifen zwischen den Streifen der ersten Gruppe bestehen, **dadurch gekennzeichnet, daß** wenigstens die Streifen in einer der erwähnten Gruppen unter Anwendung einer wasserdispersiven, wasserunlöslichen Substanz ausgebildet sind, die die Fluidtransporteigenschaften des Vlieses in Längs- und Querrichtung der Streifen beeinflußt.

6. Vlies nach Anspruch 5, bei dem die hydrophilen und/oder hydrophoben Streifen auf dem Vlies durch Aufdrucken einer Flüssigkeit aus einer Wasserdispersion oder einer Lösungsmittellösung aus der die Fluidtransporteigenschaften des Vlieses beeinflussenden Substanz ausgebildet sind.

7. Vlies nach den Ansprüchen 5-6, **dadurch gekennzeichnet, daß** das Vlies aus Polyolefinfasern, Polyestherfasern, Viskosefasern oder einer Kombination dieser Fasern gebildet ist.

8. Vlies nach den Ansprüchen 5-7, **dadurch gekennzeichnet, daß** das Grundgewicht des Vliesstoffs zwischen 10 g/m$^2$ und 150 g/m$^2$ liegt.

9. Vlies nach den Ansprüchen 5-8, **dadurch gekennzeichnet, daß** die Geschwindigkeit der Fluideindringung in Längsrichtung der Streifen das etwa 1,8-fache der Geschwindigkeit der Fluideindringung in Querrichtung der Streifen beträgt.

10. Absorbierender Wegwerfartikel, der in der Lage ist, aus dem Körper ausgetretene Fluide zu absorbieren und wenigstens aufweist:

    eine fluidundurchlässige äußere Abdeckschicht;
    eine absorbierende Schicht;
    wenigstens eine Schicht aus einem Vlies nach den Ansprüchen 5-9.

**Revendications**

1. Procédé pour diriger le transport de fluides dans une direction du plan de matériaux non tissés en feuille au moyen de bandes parallèles et continues formées dans le matériau non tissé en feuille, bandes dont au moins l'une a une aptitude au transport des fluides différente de celle de la bande adjacente, **caractérisé en ce que**, pour définir une bande ayant une aptitude au transport des fluides améliorée dans le matériau non tissé en feuille, la zone respective du matériau non tissé en feuille est traitée avec une substance insoluble dans l'eau, dispersible dans l'eau, affectant les propriétés de transport des fluides du matériau non tissé.

2. Procédé selon la revendication 1, **caractérisé en ce que** les bandes sont formées en deux groupes de bandes alternées où les bandes appartenant au même desdits groupes ont des propriétés de transport des fluides sensiblement identiques.

3. Procédé selon la revendication 2, **caractérisé en ce que** le matériau non tissé est traité avec la substance pour définir les bandes dans l'un desdits groupes de bandes, tandis que les propriétés de transport des fluides du matériau non tissé initial demeurent pour les bandes de l'autre groupe.

4. Procédé selon l'une quelconque des revendications 1 à 3, **caractérisé en ce que** les bandes ayant des propriétés de transport des fluides améliorées sont appliquées au matériau non tissé en feuille par une technologie d'impression, en particulier la technologie d'impression flexographique.

**5.** Matériau non tissé en feuille ou nappe comprenant :

un premier groupe de bandes parallèles consistant en bandes hydrophiles continues,
un second groupe de bandes consistant en bandes hydrophobes continues entre les bandes du premier groupe, **caractérisé en ce qu'**au moins les bandes dans l'un desdits groupes sont obtenues au moyen d'une substance insoluble dans l'eau, dispersible dans l'eau, affectant les propriétés de transport des fluides du matériau non tissé dans la direction allongée et dans la direction transversale des bandes.

**6.** Matériau non tissé selon la revendication 5, où lesdites bandes hydrophiles et/ou hydrophobes sont imprimées sur le matériau non tissé en feuille au moyen d'un liquide d'impression consistant en une dispersion dans l'eau, ou une solution dans un solvant de la substance affectant les propriétés de transport des fluides du matériau non tissé.

**7.** Matériau non tissé selon les revendications 5-6, **caractérisé en ce que** ledit matériau non tissé est composé de fibres de polyoléfine, de fibres de polyester, de fibres de viscose ou de combinaisons desdites fibres.

**8.** Matériau non tissé selon les revendications 5-7, **caractérisé en ce que** le grammage dudit matériau est de 10 g/m$^2$ à 150 g/m$^2$.

**9.** Matériau non tissé selon les revendications 5-8, **caractérisé en ce que** la vitesse de pénétration des fluides dans la direction allongée desdites bandes est au moins 1,8 fois supérieure à la vitesse de pénétration des fluides dans la direction transversale desdites bandes.

**10.** Article absorbant jetable capable d'absorber des liquides biologiques émis, comprenant au moins :

une couche de couverture externe imperméable aux fluides ;
une couche absorbante ;
au moins une couche constituée par le matériau non tissé selon les revendications 5-9.

Fig. 1

Fig. 2

Fig. 3

Fig. 4

Fig. 5

Fig. 6

Fig. 7